# EUROPEAN PATENT APPLICATION

(11) **EP 2 455 076 A1**
(43) Date of publication of application: **23.05.2012**
(21) Application number: 12154458.9
(22) Date of filing: 08.04.2008
(51) Int. Cl.: A61K 31/137, A61K 31/381, A61P 11/00

(54) **Compositions comprising desvenlafaxine for use in treating sleep-related breathing disorders**

(30) Priority: 09.04.2007 US 922466 P
(62) Divisional of application: 08742623.5
(71) Applicant: Sepracor Inc., Marlborough, Massachusetts 01752 (US)
(72) Inventor: Handley, Dean A., Shrewsbury, MA 01454 (US); Hardy, Larry W., Sturbridge, MA 01566 (US); Parsey, Merdad Vaziri, San Carlos, CA 94070 (US)
(74) Representative: Weber, Martin

(57) **Abstract**

Provided herein are methods of treating a sleep-related breathing disorder, such as obstructive sleep apnea, comprising the administration of O-desmethylvenlafaxine or duloxetine.

## Description

This application claims priority to U.S. Provisional Application No. 60/922,466, filed April 9, 2007, the entirety of which is incorporated herein by reference.

### 1. FIELD

Disclosed herein relate to methods and compositions for the treatment of sleep-related breathing disorders.

### 2. BACKGROUND

Over the past several years much effort has been devoted to the study of a discrete group of breathing disorders that occur primarily during sleep with consequences that may persist throughout the waking hours in the form of sleepiness, thereby manifesting itself into substantial economic loss (*e.g*., thousands of lost person-hours) or employment safety factors (*e.g*., employee inattentiveness during operation of heavy machinery). Sleep-related breathing disorders are characterized by repetitive reduction in breathing (hypopnea), periodic cessation of breathing (apnea), or a continuous or sustained reduction in ventilation.

In general, sleep apnea is defined as an intermittent cessation of airflow at the nose and mouth during sleep. By convention, apneas of at least 10 seconds in duration have been considered important, but in most individuals the apneas are 20-30 seconds in duration and may be as long as 2-3 minutes. While there is some uncertainty as to the minimum number of apneas that should be considered clinically important, by the time most individuals come to the attention of the medical community they have at least 10 to 15 events per hour of sleep.

Sleep apneas have been classified into three types: central, obstructive, and mixed. In central sleep apnea the neural drive to all respiratory muscles is transiently abolished. In obstructive sleep apneas, airflow ceases despite continuing respiratory drive because of occlusion of the oropharyngeal airway. Mixed apneas, which consist of a central apnea followed by an obstructive component, are a variant of obstructive sleep apnea. The most common type of apnea is obstructive sleep apnea.

Obstructive sleep apnea syndrome (OSAS) has been identified in as many as 24% of working adult men and 9% of similar women, with peak prevalence in the sixth decade. Habitual heavy snoring, which is an almost invariant feature of OSAS, has been described in up to 24% of middle aged men, and 14% of similarly aged women, with even greater prevalence in older subjects.

Obstructive sleep apnea syndrome's definitive event is the occlusion of the upper airway, frequently at the level of the oropharynx. The resultant apnea generally leads to a progressive-type asphyxia until the individual is briefly aroused from the sleeping state, thereby restoring airway patency and thus restoring airflow.

An important factor that leads to the collapse of the upper airway in OSAS is the generation of a critical subatmospheric pressure during the act of inspiration that exceeds the ability of the airway dilator and abductor muscles to maintain airway stability. Sleep plays a crucial role by reducing the activity of the muscles of the upper airways including the dilator and abductor muscles.

In most individuals with OSAS, the patency of the airway is also compromised structurally and is therefore predisposed to occlusion. In a minority of individuals the structural compromise is usually due to obvious anatomic abnormalities, *i.e*., adenotonsillar hypertrophy, retrognathia, or macroglossia. However, in the majority of individuals predisposed to OSAS, the structural abnormality is simply a subtle reduction in airway size, *i.e*., "pharyngeal crowding." Obesity also frequently contributes to the reduction in size seen in the upper airways. The act of snoring, which is actually a highfrequency vibration of the palatal and pharyngeal soft tissues that results from the decrease in the size of the upper airway lumen, usually aggravates the narrowing via the production of edema in the soft tissues.

The recurrent episodes of nocturnal asphyxia and of arousal from sleep that characterize OSAS lead to a series of secondary physiologic events, which in turn give rise to the clinical complications of the syndrome. The most common manifestations are neuropsychiatric and behavioral disturbances that are thought to arise from the fragmentation of sleep and loss of slow-wave sleep induced by the recurrent arousal responses. Nocturnal cerebral hypoxia also may play an important role. The most pervasive manifestation is excessive daytime sleepiness. OSAS is now recognized as a leading cause of daytime sleepiness and has been implicated as an important risk factor for such problems as motor vehicle accidents. Other related symptoms include intellectual impairment, memory loss, personality disturbances, and impotence.

The other major manifestations are cardiorespiratory in nature and are thought to arise from the recurrent episodes of nocturnal asphyxia. Most individuals demonstrate a cyclical slowing of the heart during the apneas to 30 to 50 beats per minute, followed by tachycardia of 90 to 120 beats per minute during the ventilatory phase. A small number of individuals develop severe bradycardia with asystoles of 8 to 12 seconds in duration or dangerous tachyarrhythmias, including unsustained ventricular tachycardia. OSAS also aggravates left ventricular failure in patients with underlying heart disease. This complication is most likely due to the combined effects of increased left ventricular afterload during each obstructive event, secondary to increased negative intrathoracic pressure, recurrent nocturnal hypoxemia, and chronically elevated sympathoadrenal activity.

Currently, the most common treatment for adults with sleep apnea and other sleep-related breathing disorders are mechanical forms of therapy that deliver positive airway pressure (PAP) (such as continuous positive airway pressure, CPAP). Under PAP treatment, an individual wears a tight-fitting plastic mask over the nose when sleeping. The mask is attached to a compressor, which forces air into the nose creating a positive pressure within the patient's airways. The principle of the method is that pressurizing the airways provides a mechanical "splinting" action, which prevents airway collapse and therefore, obstructive sleep apnea. Although a therapeutic response is observed in patients who undergo PAP treatment, many patients cannot tolerate the apparatus or pressure and refuse treatment. Moreover, recent covert monitoring studies clearly demonstrate that long-term compliance with PAP treatment is very poor.

A variety of upper airway and craniofacial surgical procedures have been attempted for treatment of OSAS. Adenotonsillectomy is reported to be an effective cure for OSAS in many children, but upper airway surgery is rarely curative in adult patients with OSAS. Surgical success is generally taken to be a 50% reduction in apnea incidence, and there are no useful screening methods to identify the individuals that would benefit from the surgery versus those who would not derive a benefit.

Therefore, there is a need for treatments for individuals suffering from a range of sleep-related breathing disorders. There also remains a need for a viable treatment of sleep-related breathing disorders that facilitates patient compliance.

### 3. SUMMARY

Provided herein is a method of treating one or more sleep-related breathing disorders in a patient, which comprises administering O-desmethylvenlafaxine or a pharmaceutically acceptable salt or solvate thereof.

In certain embodiments, O-desmethylvenlafaxine is enriched for either the (+) or the (-) enantiomer. In some embodiments, O-desmethylvenlafaxine is enriched for the (-) enantiomer. In some embodiments, (-)-O-desmethylvenlafaxine is substantially free of the (+) enantiomer.

Also provided herein is a method of treating one or more sleep-related breathing disorders in a patient, which comprises administering duloxetine or a pharmaceutically acceptable salt or solvate thereof.

In certain embodiments, the sleep-related breathing disorder is obstructive sleep apnea.

### 4. DETAILED DESCRIPTION

### 4.1 Methods of Treatment

Provided herein are methods of treatment with O-desmethylvenlafaxine. In certain embodiments, the therapeutic preparation may be enriched to provide predominantly one enantiomer of O-desmethylvenlafaxine. An enantiomerically enriched mixture may comprise, for example, at least 60 mol percent of one enantiomer, or more preferably at least 75, 90, 95, or 99 mol percent or more of one enantiomer. In certain embodiments, O-desmethylvenlafaxine is enriched in the (-) enantiomer. In certain embodiments, (-)-O-desmethylvenlafaxine is substantially free of the (+)-enantiomer. In certain embodiments, O-desmethylvenlafaxine is enriched in the (+) enantiomer. In certain embodiments, (+)-O-desmethylvenlafaxine is substantially free of the (-)-enantiomer. Substantially free, as the term is used herein, means that the contaminant or less desired substance makes up less than 10%, or less than 5%, or less than 4%, or less than 3%, or less than 2%, or less than 1% as compared to the amount of the compound of interest, *e.g*., in the composition or compound mixture. For example, if a composition or compound mixture contains 98 grams of the (-)-enantiomer and 2 grams of the (+)-enantiomer, it would be said to contain 98 mol percent of the (-)-enantiomer and only 2% of the (+)-enantiomer.

Provided herein is a method of treating one or more sleep-related breathing disorders in a patient, comprising administering to a patient a therapeutically effective amount of O-desmethylvenlafaxine or a pharmaceutically acceptable salt or solvate thereof.

In certain embodiments, O-desmethylvenlafaxine is enriched for either the (+) or the (-) enantiomer. In some embodiments, O-desmethylvenlafaxine is enriched for the (-) enantiomer. In some embodiments, (-)-O-desmethylvenlafaxine is substantially free of the (+) enantiomer.

In certain embodiments, O-desmethylvenlafaxine is administered conjointly with a second active agent. In certain such embodiments, the second agent is a sleep enhancer, an agent that treats excessive daytime drowsiness, an anti-obesity agent, or an agent that stabilizes respiratory drive.

In certain embodiments, O-desmethylvenlafaxine is administered conjointly with positive airway pressure treatment.

In certain embodiments, the O-desmethylvenlafaxine is administered orally, sublingually, or by oral inhalation.

In certain embodiments, the sleep-related breathing disorder is obstructive sleep apnea.

Also provided herein is a method of treating one or more sleep-related breathing disorders in a patient, comprising administering to a patient in need of such treatment a therapeutically effective amount of duloxetine, or a pharmaceutically acceptable salt or solvate thereof.

In certain embodiments, duloxetine is administered conjointly with a second active agent. In certain such embodiments, the second agent is a sleep enhancer, an agent that treats excessive daytime drowsiness, an anti-obesity agent, or an agent that stabilizes respiratory drive.

In certain embodiments, duloxetine is administered conjointly with positive airway pressure treatment.

In certain embodiments, the duloxetine is administered orally, sublingually, or by oral inhalation.

In certain embodiments, the sleep-related breathing disorder is obstructive sleep apnea.

In certain embodiments wherein O-desmethylvenlafaxine or duloxetine is administered conjointly with a sleep enhancer, the sleep enhancer may be any suitable sleep enhancer. Such sleep enhancers include, but are not limited to, eszopiclone, diphenhydramine, ramelteon, benzodiazepines (*e.g*., flurazepam, quazepam, triazolam, estazolam, and temazepam), non-benzodiazepine benzodiazepine receptor agonists (*e.g*., zolpidem or zaleplon), melatonin, gaboxadol, antihistamines (*e.g*., diphenhydramine), sedating antidepressants (*e.g*., trazodone, amitriptyline, and doxepine), and/or pharmaceutically acceptable salts or solvates thereof. In certain embodiments, the sleep enhancer is eszopiclone.

In certain embodiments wherein O-desmethylvenlafaxine or duloxetine is administered conjointly with an agent that treats excessive daytime drowsiness, the agent that treats excessive daytime drowsiness may be any suitable agent that treats excessive daytime drowsiness. Such agents that treat excessive daytime drowsiness include, but are not limited to, modafinil, caffeine, amphetamines (*e.g*., amphetamine, methamphetamine, dextroamphetamine, and levo-amphetamine), SDZ-NV1-085, mazindol, methylphenidate, and/or pharmaceutically acceptable salts or solvates thereof.

In certain embodiments wherein O-desmethylvenlafaxine or duloxetine is administered conjointly with an anti-obesity agent, the anti-obesity agent may be any suitable anti-obesity agent. Such anti-obesity agents include, but are not limited to, orlistat, sibutramine, phendimetrazine, phentermine, diethylpropion, benzphetamine, mazindol, dextroamphetamine, rimonabant, cetilistat, GT 389-255, APD356, pramlintide/AC137, PYY3-36, AC 162352/PYY3-36, oxyntomodulin, TM 30338, AOD 9604, oleoyl-estrone, bromocriptine, ephedrine, leptin, pseudoephedrine, and/or pharmaceutically acceptable salts or solvates thereof.

In certain embodiments wherein O-desmethylvenlafaxine or duloxetine is administered conjointly with an agent that stabilizes respiratory drive, the agent that stabilizes respiratory drive is selected from any suitable agent that stabilizes respiratory drive. Such agents that stabilizes respiratory drive include, but are not limited to, topiramate, amantadine, bupropion, modafinil, r-modafinil, SDZ-NV1-085, 5-HT1A agonists (*e.g*., buspirone, gepirone, alnespirone, and ORG 13011), zonisamide, and/or pharmaceutically acceptable salts or solvates thereof.

Administration of an active agent provided herein, or a pharmaceutically acceptable salt or solvate thereof, and the second active agents to a patient can occur simultaneously or sequentially by the same or different routes of administration. The suitability of a particular route of administration employed for a particular active agent will depend on the active agent itself (*e.g*., whether it can be administered orally without decomposing prior to entering the blood stream) and the disease being treated. One of administration for compounds provided herein is oral. Routes of administration for the second active agents or ingredients are known to those of ordinary skill in the art. *See*, *e.g*., Physicians' Desk Reference (60th ed., 2006).

Both (-)-O-desmethylvenlafaxine and duloxetine exhibit inhibition of both serotonin (5-HT) and norepinephrine (NE) reuptake. Without being limited by a particular theory, his dual inhibition results in increased levels of 5-HT and NE in the central nervous system. Without being limited by a particular theory, this dual-inhibitory activity may mediate the utility of these compounds for the treatment of sleep-related breathing disorders, such as obstructive sleep apnea. Further without being limited by a particular theory, elevated levels of 5-HT and NE in the brain results in the stimulation of nerves that regulate the tone of upper airway muscles, such as cranial nerve XII (hypoglossal nerve), which in turn controls the genioglossus muscle. Without being limited by a particular theory, regulation of the tone of these muscles would maintain the patency of the upper airway during sleep, thereby preventing apneic episodes.

Also provided herein are uses of metabolites of the compounds disclosed herein (*e.g*., duloxetine, (±)-O-desmethylvenlafaxine, (+)-O-desmethylvenlafaxine, or (-)-O-desmethylvenlafaxine). The term "metabolite" is intended to encompass compounds that are produced by metabolism of the parent compound under normal physiological conditions. For example, an N-methyl group may be cleaved to produce the corresponding N-desmethyl metabolite. Specific metabolites include those that exhibit similar activity to their parent compound (*e.*g., metabolites that are suitable for the treatment of one or more sleep-related breathing disorders in a patient).

Doses of a compound provided herein, or a pharmaceutically acceptable salt or solvate thereof, vary depending on factors such as: specific indication to be treated; age and condition of a patient; and amount of second active agent used, if any; routes of administration; and result sought by the treatment. Generally, a compound provided herein, or a pharmaceutically acceptable salt or solvate thereof, may be used in an amount of from about 0.001 mg to about 1000 mg per day, and can be adjusted in a conventional fashion (*e.g*., the same amount administered each day of the treatment, prevention or management period), in cycles (*e.g*., one week on, one week off), or in an amount that increases or decreases over the course of treatment.

Any suitable routes of administration may be used for the methods of treatment provided herein. Such routes include, but are not limited to, oral, mucosal (*e.g*., nasal, sublingual, vaginal, buccal, or rectal), parenteral (*e.g*., subcutaneous, intravenous, bolus injection, intramuscular, or intraarterial), topical (*e.g*., eye drops or other ophthalmic preparations), transdermal or transcutaneous administration.

Compounds disclosed herein (*e.g*., duloxetine, (±)-O-desmethylvenlafaxine, (+)-O-desmethylvenlafaxine, or (-)-O-desmethylvenlafaxine) are administered to a patient. In certain embodiments, the patient is a mammal such as a human, or a non-human mammal.

### 4.2 Pharmaceutical Compositions and Dosage Forms

When administered to a patient, the compound disclosed herein (*e.g*., duloxetine, (±)-O-desmethylvenlafaxine, (+)-O-desmethylvenlafaxine, or (-)-O-desmethylvenlafaxine) and/or another active agent can be administered as a pharmaceutical composition. Pharmaceutical compositions can be used in the preparation of individual, single unit dosage forms. Pharmaceutical compositions and dosage forms provided herein comprise a compound provided herein, or a pharmaceutically acceptable salt or solvate thereof. Pharmaceutical compositions and dosage forms can further comprise one or more excipients.

Examples of dosage forms include, but are not limited to: tablets; caplets; capsules, such as soft elastic gelatin capsules; cachets; troches; lozenges; dispersions; suppositories; powders; aerosols (*e.g*., nasal sprays or inhalers); gels; liquid dosage forms suitable for oral or mucosal administration to a patient, including suspensions (*e.g*., aqueous or non-aqueous liquid suspensions, oil-in-water emulsions, or a water-in-oil liquid emulsions), solutions, and elixirs; liquid dosage forms suitable for parenteral administration to a patient; eye drops or other ophthalmic preparations suitable for topical administration; and sterile solids (*e.g*., crystalline or amorphous solids) that can be reconstituted to provide liquid dosage forms suitable for parenteral administration to a patient.

The composition, shape, and type of dosage forms will typically vary depending on their use. For example, a dosage form used in the acute treatment of a disease may contain larger amounts of one or more of the active ingredients it comprises than a dosage form used in the chronic treatment of the same disease. Similarly, a parenteral dosage form may contain smaller amounts of one or more of the active ingredients it comprises than an oral dosage form used to treat the same disease. These and other ways in which specific dosage forms are used will vary from one another will be readily apparent to those skilled in the art. *See, e.g*., Remington's Pharmaceutical Sciences, 18th ed., Mack Publishing, Easton PA (1990).

Pharmaceutically acceptable carriers are well known in the art and include, for example, aqueous solutions such as water or physiologically buffered saline or other solvents or vehicles such as glycols, glycerol, oils such as olive oil or injectable organic esters. In a preferred embodiment, when such pharmaceutical compositions are for human administration, the aqueous solution is pyrogen-free, or substantially pyrogen-free. The excipients can be chosen, for example, to effect delayed release of an agent or to selectively target one or more cells, tissues or organs. The pharmaceutical composition can be in dosage unit form such as tablet, capsule, sprinkle capsule, granule, powder, syrup, suppository, injection or the like. The composition can also be present in a transdermal delivery system, *e.g*., a skin patch.

A pharmaceutically acceptable carrier can contain physiologically acceptable agents that act, for example, to stabilize or to increase the absorption of a compound such as duloxetine or O-desmethylvenlafaxine. Such physiologically acceptable agents include, for example, carbohydrates, such as glucose, sucrose or dextrans, antioxidants, such as ascorbic acid or glutathione, chelating agents, low molecular weight proteins or other stabilizers or excipients. The choice of a pharmaceutically acceptable carrier, including a physiologically acceptable agent, depends, for example, on the route of administration of the composition. The pharmaceutical composition (preparation) also can be a liposome or other polymer matrix, which can have incorporated therein, for example, an active agent. Liposomes, for example, which consist of phospholipids or other lipids, are nontoxic, physiologically acceptable and metabolizable carriers that are relatively simple to make and administer.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically acceptable carrier" as used herein means a pharmaceutically acceptable material, composition or vehicle, such as a liquid or solid filler, diluent, excipient, solvent or encapsulating material. Each carrier must be "acceptable" in the sense of being compatible with the other ingredients of the formulation and not injurious to the patient. Some examples of materials which can serve as pharmaceutically acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations.

A pharmaceutical composition (preparation) containing a compound disclosed herein (*e.g*., duloxetine, (±)-O-desmethylvenlafaxine, (+)-O-desmethylvenlafaxine, or (-)-O-desmethylvenlafaxine) can be administered to a subject by any of a number of routes of administration including, for example, orally (for example, drenches as in aqueous or non-aqueous solutions or suspensions, tablets, boluses, powders, granules, pastes for application to the tongue); sublingually; anally, rectally or vaginally (for example, as a pessary, cream or foam); parenterally (including intramuscularly, intravenously, subcutaneously or intrathecally as, for example, a sterile solution or suspension); nasally; intraperitoneally; subcutaneously; transdermally (for example as a patch applied to the skin); and topically (for example, as a cream, ointment or spray applied to the skin). The compound may also be formulated for oral inhalation. In certain embodiments, a compound disclosed herein (*e.g*., duloxetine, (±)-O-desmethylvenlafaxine, (-)-O-desmethylvenlafaxine, or (+)-O-desmethylvenlafaxine) may be simply dissolved or suspended in sterile water. Details of appropriate routes of administration and compositions suitable for same can be found in, for example, U.S. Pat. Nos. 6,110,973, 5,763,493, 5,731,000, 5,541,231, 5,427,798, 5,358,970 and 4,172,896, as well as in patents cited therein. The most preferred route of administration is the oral route.

The formulations may conveniently be presented in unit dosage form and may be prepared by any methods well known in the art of pharmacy. The amount of active ingredient which can be combined with a carrier material to produce a single dosage form will vary depending upon the host being treated, the particular mode of administration. The amount of active ingredient that can be combined with a carrier material to produce a single dosage form will generally be that amount of the compound which produces a therapeutic effect. Generally, out of one hundred percent, this amount will range from about 1 percent to about ninety-nine percent of active ingredient, preferably from about 5 percent to about 70 percent, most preferably from about 10 percent to about 30 percent.

Methods of preparing these formulations or compositions include bringing into association a compound provided herein with the carrier and, optionally, one or more accessory ingredients. In general, the formulations are prepared by uniformly and intimately bringing into association a compound provided herein with liquid carriers, or finely divided solid carriers, or both, and then, if necessary, shaping the product.

Formulations suitable for oral administration may be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a compound provided herein as an active ingredient. A compound disclosed herein may also be administered as a bolus, electuary or paste.

In solid dosage forms for oral administration (capsules, tablets, pills, dragees, powders, granules and the like), the active ingredient is mixed with one or more pharmaceutically acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, cetyl alcohol and glycerol monostearate; (8) absorbents, such as kaolin and bentonite clay; (9) lubricants, such a talc, calcium stearate, magnesium stearate, solid polyethylene glycols, sodium lauryl sulfate, and mixtures thereof; and (10) coloring agents. In the case of capsules, tablets and pills, the pharmaceutical compositions may also comprise buffering agents. Solid compositions of a similar type may also be employed as fillers in soft and hard-filled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered compound moistened with an inert liquid diluent.

The tablets, and other solid dosage forms of the pharmaceutical compositions, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be sterilized by, for example, filtration through a bacteria-retaining filter, or by incorporating sterilizing agents in the form of sterile solid compositions that can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions that can be used include, but are not limited to, polymeric substances and waxes. The active ingredient can also be in micro-encapsulated form, if appropriate, with one or more of the above-described excipients.

Liquid dosage forms for oral administration of the active agents include, but are not limited to, pharmaceutically acceptable emulsions, microemulsions, solutions, suspensions, syrups and elixirs. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, coloring, perfuming and preservative agents.

Suspensions, in addition to the active compounds, may contain suspending agents such as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Formulations for rectal, vaginal, or urethral administration may be presented as a suppository, which may be prepared by mixing one or more active agents with one or more suitable nonirritating excipients or carriers comprising, for example, cocoa butter, polyethylene glycol, a suppository wax or a salicylate, and which is solid at room temperature, but liquid at body temperature and, therefore, will melt in the rectum or vaginal cavity and release the active compound.

Alternatively or additionally, compositions can be formulated for delivery via a catheter, stent, wire, or other intraluminal device. Delivery via such devices may be especially useful for delivery to the bladder, urethra, ureter, rectum, or intestine.

Formulations which are suitable for vaginal administration also include pessaries, tampons, creams, gels, pastes, foams or spray formulations containing such carriers as are known in the art to be appropriate.

Dosage forms for the topical or transdermal administration of a compound disclosed herein include powders, sprays, ointments, pastes, creams, lotions, gels, solutions, patches and inhalants. The active compound may be mixed under sterile conditions with a pharmaceutically acceptable carrier, and with any preservatives, buffers, or propellants that may be required.

The ointments, pastes, creams and gels may contain, in addition to an active compound, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to a compound provided herein, excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

Transdermal patches have the added advantage of providing controlled delivery of a compound to the body. Such dosage forms can be made by dissolving or dispersing the compound in the proper medium. Absorption enhancers can also be used to increase the flux of the compound across the skin. The rate of such flux can be controlled by either providing a rate controlling membrane or dispersing the compound in a polymer matrix or gel.

Ophthalmic formulations, eye ointments, powders, solutions and the like, are also contemplated as being within the scope.

The phrases "parenteral administration" and "administered parenterally" as used herein mean modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal and intrasternal injection and infusion.

Pharmaceutical compositions suitable for parenteral administration comprise one or more active agents in combination with one or more pharmaceutically acceptable sterile isotonic aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

Examples of suitable aqueous and nonaqueous carriers that may be employed in the pharmaceutical compositions include, but are not limited to, water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents that delay absorption such as aluminum monostearate and gelatin.

In some cases, in order to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution, which, in turn, may depend upon crystal size and crystalline form. Alternatively, delayed absorption of a parenterally administered drug form is accomplished by dissolving or suspending the drug in an oil vehicle.

Injectable depot forms are made by forming microencapsuled matrices of the subject compounds in biodegradable polymers such as polylactide-polyglycolide. Depending on the ratio of drug to polymer, and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions that are compatible with body tissue.

When the compounds disclosed herein are administered as pharmaceuticals, to humans and animals, they can be given *per se* or as a pharmaceutical composition containing, for example, 0.1 to 99.5% (more preferably, 0.5 to 90%) of active ingredient in combination with a pharmaceutically acceptable carrier.

Methods of introduction may also be provided by rechargeable or biodegradable devices. Various slow release polymeric devices have been developed and tested *in vivo* in recent years for the controlled delivery of drugs, including proteinaceous biopharmaceuticals. A variety of biocompatible polymers (including hydrogels), including both biodegradable and non-degradable polymers, can be used to form an implant for the sustained release of a compound at a particular target site.

Actual dosage levels of the active ingredients in the pharmaceutical compositions may be varied so as to obtain an amount of the active ingredient that is effective to achieve the desired therapeutic response for a particular patient, composition, and mode of administration, without being toxic to the patient.

The selected dosage level will depend upon a variety of factors including the activity of the particular active agent employed, or the ester, salt or amide thereof, the route of administration, the time of administration, the rate of excretion of the particular compound being employed, the duration of the treatment, other drugs, compounds and/or materials used in combination with the particular compound employed, the age, sex, weight, condition, general health and prior medical history of the patient being treated, and like factors well known in the medical arts.

A physician or veterinarian having ordinary skill in the art can readily determine and prescribe the effective amount of the pharmaceutical composition required. For example, the physician or veterinarian could start doses of the agents employed in the pharmaceutical composition at levels lower than that required in order to achieve the desired therapeutic effect and gradually increase the dosage until the desired effect is achieved.

In general, a suitable daily dose of an active agent will be that amount of the compound that is the lowest dose effective to produce a therapeutic effect. Such an effective dose will generally depend upon the factors described above.

If desired, the effective daily dose of the active compound may be administered as one, two, three, four, five, six or more sub-doses administered separately at appropriate intervals throughout the day, optionally, in unit dosage forms. In certain embodiments, the active compound may be administered two or three times daily. In preferred embodiments, the active compound will be administered once daily.

The patient receiving this treatment is any animal in need, including primates, in particular humans, and other mammals such as equines, cattle, swine and sheep; and poultry and pets in general.

In certain embodiments, compounds disclosed herein (*e.g*., duloxetine, (±)-O-desmethylvenlafaxine, (+)-O-desmethylvenlafaxine, or (-)-O-desmethylvenlafaxine) may be used alone or conjointly administered with another treatment or type of therapeutic agent. As used herein, the phrase "conjoint administration" refers to any form of administration of two or more different therapeutic compounds such that the second compound is administered while the previously administered therapeutic compound is still effective in the body (*e.g*., the two compounds are simultaneously effective in the patient, which may include synergistic effects of the two compounds). For example, the different therapeutic compounds can be administered either in the same formulation or in a separate formulation, either concomitantly or sequentially. Thus, an individual who receives such treatment can benefit from a combined effect of different therapeutic compounds. In addition, "conjoint administration" refers to any form of administration of compounds provided herein (*e.g*., duloxetine, (±)-O-desmethylvenlafaxine, (+)-O-desmethylvenlafaxine, or (-)-O-desmethylvenlafaxine) in combination with a second, non-pharmaceutical treatment (*e.g*., positive airway pressure treatment) that occurs while the compound provided herein is effective in the body (*e.g*., the two mechanisms of treatment are simultaneously effective in the patient, which may include synergistic effects of the two mechanisms). Thus, an individual who receives such treatment can benefit from a combined effect of the different therapies.

It is contemplated that compounds disclosed herein (*e.g*., duloxetine, (±)-O-desmethylvenlafaxine, (+)-O-desmethylvenlafaxine, or (-)-O-desmethylvenlafaxine) will be administered to a subject (*e.g*., a mammal, preferably a human) in a therapeutically effective amount (dose). By "therapeutically effective amount" is meant the concentration of a compound that is sufficient to elicit the desired therapeutic effect (*e.g*., treatment or prevention of a sleep-related breathing disorder such as obstructive sleep apnea). It is generally understood that the effective amount of the compound will vary according to the weight, sex, age, and medical history of the subject. Other factors which influence the effective amount may include, but are not limited to, the severity of the patient's condition, the disorder being treated, the stability of the compound, and, if desired, another type of therapeutic agent being administered with an active agent. A larger total dose can be delivered by multiple administrations of the agent. Methods to determine efficacy and dosage are known to those skilled in the art (Isselbacher et al. (1996) Harrison's Principles of Internal Medicine 13 ed., 1814-1882, herein incorporated by reference).

Also provided herein are the pharmaceutically acceptable salts or solvates of compounds provided herein (*e.g*., duloxetine, (±)-O-desmethylvenlafaxine, (+)-O-desmethylvenlafaxine, or (-)-O-desmethylvenlafaxine). Without being limited by a particular theory, since duloxetine and venlafaxine are amines, they are basic in nature and accordingly react with any number of inorganic and organic acids to form pharmaceutically acceptable acid addition salts. Acids commonly employed to form such salts include inorganic acids such as hydrochloric, hydrobromic, hydriodic, sulfuric and phosphoric acid, as well as organic acids such as para-toluenesulfonic, methanesulfonic, oxalic, para-bromophenylsulfonic, carbonic, succinic, citric, benzoic and acetic acid, and related inorganic and organic acids. Such pharmaceutically acceptable salts thus include sulfate, pyrosulfate, bisulfate, sulfite, bisulfite, phosphate, monohydrogenphosphate, dihydrogenphosphate, metaphosphate, pyrophosphate, chloride, bromide, iodide, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, dinitrobenzoate, hydroxybenzoate, methoxybenzoate, phthalate, terephthalate, sulfonate, xylenesulfonate, phenylacetate, phenylpropionate, phenylbutyrate, citrate, lactate, .beta.-hydroxybutyrate, glycollate, maleate, tartrate, methanesulfonate, propanesulfonates, naphthalene-1-sulfonate, naphthalene-2-sulfonate, mandelate, hippurate, gluconate, lactobionate, and the like salts.

The pharmaceutically acceptable acid addition salts of compounds provided herein (*e.g*., duloxetine, (±)-O-desmethylvenlafaxine, (+)-O-desmethylvenlafaxine, or (-)-O-desmethylvenlafaxine) can also exist as various solvates, such as with water, methanol, ethanol, dimethylformamide, and the like. Mixtures of such solvates can also be prepared. The source of such solvate can be from the solvent of crystallization, inherent in the solvent of preparation or crystallization, or adventitious to such solvent.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Also provided herein is a kit comprising:
a) one or more single dosage forms comprising a compound provided herein (*e.g*., duloxetine, (±)-O-desmethylvenlafaxine, (-)-O-desmethylvenlafaxine, or (+)-O-desmethylvenlafaxine) and a pharmaceutically acceptable excipient; and
b) instructions for administering the single dosage forms for the treatment of a sleep-related breathing disorder, such as obstructive sleep apnea.

Also provided herein is a kit comprising:
a) a first pharmaceutical formulation comprising a compound provided herein (*e.g*., duloxetine, (±)-O-desmethylvenlafaxine, (-)-O-desmethylvenlafaxine, or (+)-O-desmethylvenlafaxine);
b) a second pharmaceutical formulation comprising at least one of the following: a sleep enhancer, an agent that treats excessive daytime drowsiness, an anti-obesity agent, or an agent that stabilizes respiratory drive; and
c) instructions for the administration of the first and second pharmaceutical formulations.

In certain embodiments, provided herein is a method for conducting a pharmaceutical business, by manufacturing a formulation of a compound provided herein (*e.g*., duloxetine, (±)-O-desmethylvenlafaxine, (-)-O-desmethylvenlafaxine, or (+)-O-desmethylvenlafaxine) or a kit as described herein, and marketing to healthcare providers the benefits of using the formulation or kit in the treatment or prevention of a sleep-related breathing disorder, such as obstructive sleep apnea.

In certain embodiments, provided herein is a method for conducting a pharmaceutical business, by manufacturing a formulation of a compound provided herein (*e.g*., duloxetine, (±)-O-desmethylvenlafaxine, (-)-O-desmethylvenlafaxine, or (+)-O-desmethylvenlafaxine) to be administered conjointly with a sleep enhancer, an agent that treats excessive daytime drowsiness, an anti-obesity agent, or an agent that stabilizes respiratory drive, or a kit as described herein, and marketing to healthcare providers the benefits of using the formulation or kit in the treatment or prevention of a sleep-related breathing disorder, such as obstructive sleep apnea.

In certain embodiments, provided herein is a method for conducting a pharmaceutical business, by providing a distribution network for selling a formulation of a compound provided herein (*e.g*., duloxetine, (±)-O-desmethylvenlafaxine, (-)-O-desmethylvenlafaxine, or (+)-O-desmethylvenlafaxine) or kit as described herein, and providing instruction material to patients or physicians for using the formulation to treat or prevent a sleep-related breathing disorder, such as obstructive sleep apnea.

In certain embodiments, provided herein is a method for conducting a pharmaceutical business, by providing a distribution network for selling a formulation of a compound provided herein (*e.g*., duloxetine, (±)-O-desmethylvenlafaxine, (-)-O-desmethylvenlafaxine, or (+)-O-desmethylvenlafaxine) to be administered conjointly with a sleep enhancer, an agent that treats excessive daytime drowsiness, an anti-obesity agent, or an agent that stabilizes respiratory drive, or kit as described herein, and providing instruction material to patients or physicians for using the formulation to treat or prevent a sleep-related breathing disorder, such as obstructive sleep apnea.

In certain embodiments, provided herein is a method for conducting a pharmaceutical business, by determining an appropriate formulation and dosage of a compound provided herein (*e.g*., duloxetine, (±)-O-desmethylvenlafaxine, (-)-O-desmethylvenlafaxine, or (+)-O-desmethylvenlafaxine) to be administered for the treatment or prevention of a sleep-related breathing disorder, such as obstructive sleep apnea, conducting therapeutic profiling of identified formulations for efficacy and toxicity in animals, and providing a distribution network for selling an identified preparation as having an acceptable therapeutic profile. In certain embodiments, the method further includes providing a sales group for marketing the preparation to healthcare providers.

In certain embodiments, provided herein is a method for conducting a pharmaceutical business, by determining an appropriate formulation and dosage of a compound provided herein (*e.g*., duloxetine, (±)-O-desmethylvenlafaxine, (-)-O-desmethylvenlafaxine, or (+)-O-desmethylvenlafaxine) to be administered conjointly with a sleep enhancer, an agent that treats excessive daytime drowsiness, an anti-obesity agent, or an agent that stabilizes respiratory drive in the treatment or prevention of a sleep-related breathing disorder, such as obstructive sleep apnea, conducting therapeutic profiling of identified formulations for efficacy and toxicity in animals, and providing a distribution network for selling an identified preparation as having an acceptable therapeutic profile. In certain embodiments, the method further includes providing a sales group for marketing the preparation to healthcare providers.

In certain embodiments, provided herein is a method for conducting a pharmaceutical business by determining an appropriate formulation and dosage of a compound provided herein (*e.g*., duloxetine, (±)-O-desmethylvenlafaxine, (-)-O-desmethylvenlafaxine, or (+)-O-desmethylvenlafaxine) to be administered for the treatment or prevention of a sleep-related breathing disorder, such as obstructive sleep apnea, and licensing, to a third party, the rights for further development and sale of the formulation.

In certain embodiments, provided herein is a method for conducting a pharmaceutical business by determining an appropriate formulation and dosage of a compound provided herein (*e.g*., duloxetine, (±)-O-desmethylvenlafaxine, (-)-O-desmethylvenlafaxine, or (+)-O-desmethylvenlafaxine) to be administered conjointly with a sleep enhancer, an agent that treats excessive daytime drowsiness, an anti-obesity agent, or an agent that stabilizes respiratory drive in the treatment or prevention of a sleep-related breathing disorder, such as obstructive sleep apnea, and licensing, to a third party, the rights for further development and sale of the formulation.

The term "healthcare providers" refers to individuals or organizations that provide healthcare services to a person, community, etc. Examples of "healthcare providers" include doctors, hospitals, continuing care retirement communities, skilled nursing facilities, subacute care facilities, clinics, multispecialty clinics, freestanding ambulatory centers, home health agencies, and HMO's.

As used herein, a therapeutic that "prevents" a disorder or condition refers to a compound that, in a statistical sample, reduces the occurrence of the disorder or condition in the treated sample relative to an untreated control sample, or delays the onset or reduces the severity of one or more symptoms of the disorder or condition relative to the untreated control sample.

The term "treating" includes prophylactic and/or therapeutic treatments. The term "prophylactic or therapeutic" treatment is art-recognized and includes administration to the host of one or more of the subject compositions. If it is administered prior to clinical manifestation of the unwanted condition (*e.g*., disease or other unwanted state of the host animal) then the treatment is prophylactic, (*i.e*., it protects the host against developing the unwanted condition), whereas if it is administered after manifestation of the unwanted condition, the treatment is therapeutic, (*i.e*., it is intended to diminish, ameliorate, or stabilize the existing unwanted condition or side effects thereof).

### 5. EXAMPLES

The compounds provided herein were tested for their inhibition of functional uptake of 5-HT, NE, or DA, in synaptosomes prepared from rat whole brain, hypothalamus, or corpus striatum, respectively, or in whole cells cultured *in vitro* expressing the cloned human transporters for 5-HT, NE, or DA. The concentrations of radioligand employed in the assays were at or below the K_{M} for the monoamine substrates with their cognate transporters. Compounds were tested at 5 to 10 different concentrations in duplicate in order to obtain full inhibition curves. IC₅₀ values (concentration inhibiting control activity by 50%) were then determined by nonlinear regression analysis of the inhibition curves.

### 5.1 Serotonin Functional Uptake Assay for Rat Reuptake Transporter

Quantification of 5-HT uptake was performed using synaptosomes isolated in a 0.32M sucrose buffer from a male Wistar rat cortex. The uptake of radiolabelled 5-HT by synaptosomes (100 µg of proteins/point) was allowed by incubating them in a well for 15 min at 37 °C in presence of test compounds and [³H]5-hydroxytryptamine (0.1 µCi/point).

Synaptosomes and [³H]5-hydroxytryptamine were prepared in a Krebs buffer pH 7.4 containing 25 mM NaHCO₃, 11 mM glucose and 50 µM ascorbic acid. This incubation buffer was oxygenated during 5 minutes before incubation. Basal control was incubated for 15 minutes at 4 °C in order to avoid any uptake. Following this incubation the uptake was stopped by filtration through a unifilter 96-wells GFB Packard plate washed with Krebs buffer containing 25 mM NaHCO₃ in order to eliminate the free [³H]5-hydroxytryptamine. The radioactivity associated to the synaptosomes retained on the unifilter corresponding to the uptake was then measured with a microplate scintillation counter (Topcount, Packard) using a scintillation fluid. Nonspecific binding was measured in the presence of an excess of cold, unlabeled ligand. Specific binding was obtained by subtracting nonspecific binding from total binding.

The reference compound was imipramine tested at 10 concentrations ranging from 10⁻¹¹ M to 10⁻⁵ M in order to obtain an IC₅₀ value. *See, e.g*., Perovics and Müller, "Pharmacological profile of hypericum extract: effect on serotonin uptake by postsynaptic receptors," Arzeim. Forsch./Drug Res., 45:1145-1148 (1995).

Venlafaxine, racemic O-desmethylvenlafaxine, (+)-O-desmethylvenlafaxine, and (-)-O-desmethylvenlafaxine were evaluated in this assay. The results are displayed in Table 1.

### 5.2 Serotonin Functional Uptake Assay for Human Reuptake Transporter

Inhibition of human serotonin reuptake transporter was assayed using the recombinant human serotonin transporter expressed in HEK-293 cells using a published method (Gu H, Wall S, Rudnick G. Stable expression of biogenic amine transporters reveals differences in inhibitor sensitivity, kinetics, and ion dependence. J Biol Chem. 269 (10): 7124-7130, 1994)). Human serotonin transporter expressed in HEK-293 cells were plated before the assay. Test compound and/or vehicle was preincubated with cells (5 x 10⁵/ml) in modified Tris-HEPES buffer pH 7.1 for 20 minutes at 25°C and 65 nM [³H]Serotonin was then added for an additional 10 minute incubation period. Bound cells were filtered and the amount of tritium taken into cells is counted using a liquid scintillation counter to determine [³H]Serotonin uptake. Reduction of [³H]Serotonin uptake by 50 percent or more (≥50%) relative to 10 µM fluoxetine indicates significant inhibitory activity. Compounds were screened at 10, 1, 0.1, 0.01 and 0.001 µM. The reference compound for the assay was fluoxetine, for which the IC₅₀ value of 7.1 nM was obtained.

Venlafaxine, racemic O-desmethylvenlafaxine, (+)-O-desmethylvenlafaxine, (-)-O-desmethylvenlafaxine, and duloxetine were evaluated in this assay. The results are displayed in Table 1.

### 5.3 Dopamine Functional Uptake Assay for Rat Reuptake Transporter

Quantification of dopamine uptake was performed using synaptosomes isolated in a 0.32 M sucrose buffer from a male Wistar rat striatum. The uptake of radiolabelled dopamine by synaptosomes (20 µg of proteins/point) was allowed by incubating them for 15 minutes at 37 °C in the presence of test compounds and [³H]-dopamine (0.1 µCi/point). The experiment was performed in a deep well.

Synaptosomes and [³H]-dopamine were prepared in a Krebs buffer pH 7.4 containing 25 mM NaHCO₃, 11 mM glucose and 50 µM ascorbic acid. This incubation buffer was oxygenated for 5 minutes before incubation. Basal control was incubated for 15 minutes at 4 °C in order to avoid any uptake. Following this incubation, the uptake was stopped by filtration through a unifilter 96-wells GFB Packard plate washed with Krebs buffer containing 25 mM NaHCO₃ in order to eliminate free [³H]-dopamine. The radioactivity associated to the synaptosomes retained onto the unifilter corresponding to the uptake was then measured with a microplate scintillation counter (Topcount, Packard) using a scintillation fluid.

The reference compound GRB 12909 was tested at 8 concentrations ranging from 10⁻¹¹ M to 10⁻⁶ M in order to obtain an IC₅₀ value. See, Jankowsky et al., "Characterization of sodium-dependent [3H]GBR-12935 binding in brain: a radioligand for selective labeling of the dopamine transport complex," J. Neurochem, 46:1272-1276 (1986).

Venlafaxine, racemic O-desmethylvenlafaxine, (+)-O-desmethylvenlafaxine, and (-)-O-desmethylvenlafaxine were evaluated in this assay. The results are displayed in Table 1.

### 5.4 Dopamine Functional Uptake Assay for Human Reuptake Transporter

Inhibition of human dopamine reuptake transporter was assayed using the recombinant human dopamine transporter expressed in CHO-K1 cells using a published method (Pristupa, Z.B., Wilson, J.M., Hoffman, B.J., Kish, S.J. and Niznik, H.B. Pharmacological heterogeneity of the cloned and native human dopamine transporter: disassociation of [3H]GBR12,935 binding. Mol. Pharmacol. 45: 125-135, 1994). Human recombinant dopamine transporter expressed in CHO-K1 cells were plated before the assay. Test compound and/or vehicle was preincubated with cells (4 x 10⁵/ml) in modified Tris-HEPES buffer pH 7.1 for 20 minutes at 25°C and 50 nM [³H]Dopamine was then added for an additional 10 minute incubation period. A lysate was obtained from solubilized cells and the amount of tritium in the lysate was measured using a liquid scintillation counter to determine [³H]Dopamine uptake. Reduction of [³H]Dopamine uptake by 50 percent or more (≥50%) relative to 10 µM nomifensine indicates significant inhibitory activity. Compounds were screened at 10, 1, 0.1, 0.01 and 0.001 µM. The reference compound for the assay was nomifensine, for which the IC₅₀ value of 11 nM was obtained.

Venlafaxine, racemic O-desmethylvenlafaxine, (+)-O-desmethylvenlafaxine, (-)-O-desmethylvenlafaxine, and duloxetine were evaluated in this assay. The results are displayed in Table 1.

### 5.5 Norepinephrine Functional Uptake Assay For Rat Reuptake Transporter

Quantification of norepinephrine uptake was performed using synaptosomes isolated in a 0.32 M sucrose buffer from a male Wistar rat hypothalamus. The uptake of radiolabelled norepinephrine by synaptosomes (100 µg of proteins/point) was allowed by incubating them for 20 minutes at 37 °C in presence of test compounds and [³H]-norepinephrine (0.1 µCi/point). The experiment was performed in a deep well.

Synaptosomes and [³H]-norepinephrine were prepared in a Krebs buffer pH 7.4 containing 25 mM NaHCO₃, 11 mM glucose and 50 µM ascorbic acid. This incubation buffer was oxygenated for 5 minutes before incubation. Basal control was incubated for 20 minutes at 4 °C in order to avoid any uptake. Following this incubation, the uptake was stopped by filtration through a unifilter 96-wells GFB Packard plate washed with Krebs buffer containing 25 mM NaHCO₃ in order to eliminate the free [³H]-norepinephrine. The radioactivity associated to the synaptosomes retained onto the unifilter corresponding to the uptake was then measured with a microplate scintillation counter (Topcount, Packard) using a scintillation fluid.

The reference compound was protriptyline tested at 13 concentrations ranging from 10⁻¹¹ M to 10⁻⁵ M in order to obtain an IC₅₀ value. See, Perovics and Muller, "Pharmacological profile of hypericum extract: effect on serotonin uptake by postsynaptic receptors," Arzeim. Forsch./Drug Res., 45:1145-1148 (1995).

Venlafaxine, racemic O-desmethylvenlafaxine, (+)-O-desmethylvenlafaxine, and (-)-O-desmethylvenlafaxine were evaluated in this assay. The results are displayed in Table 1.

### 5.6 Norepinephrine Functional Uptake Assay for Human Reuptake Transporter

Inhibition of human norepinephrine reuptake transporter was assayed using the recombinant human norepinephrine transporter expressed in MDCK cells using a published method (Galli A, DeFelice LJ, Duke BJ, Moore KR, Blakely RD. Sodium dependent norepinephrine-induced currents in norepinephrine-transporter-transfected HEK-293 cells blocked by cocaine and antidepressants. J Exp Biol. 198: 2197-2212, 1995). The cells were plated one day before the assay. Test compound and/or vehicle was preincubated with cells (2 x 10⁵/ml) in modified Tri-HEPES buffer pH 7.1 for 20 minutes at 25°C and 25 nM [³H]Norepinephrine was then added for an additional 10 minute incubation period. A lysate was obtained from solubilized cells and the amount of tritium in the lysate was measured using a liquid scintillation counter to determine [³H]Norepinephrine uptake. Reduction of [³H]Norepinephrine uptake by 50 percent or more (≥50%) relative to 10 µM desipramine indicates significant inhibitory activity. Compounds were screened at 10, 1, 0.1, 0.01 and 0.001 µM. The reference compound for the assay was desipramine, for which the IC50 value of 1.9 nM was obtained.

Venlafaxine, racemic O-desmethylvenlafaxine, (+)-O-desmethylvenlafaxine, (-)-O-desmethylvenlafaxine, and duloxetine were evaluated in this assay. The results are displayed in Table 1.

**Table 1: Inhibition of Monoamine Uptake by Assays in Rat and Human Monoamine Transporters [IC₅₀ (nM)]**

| | **Rat** | | | **Human** | | |
|---|---|---|---|---|---|---|
| **Compound** | **5-HT** | **NE** | **DA** | **5-HT** | **NE** | **DA** |
| Venlafaxine | 47 | 360 | 7,000 | 27 | 185 | 4,680 |
| Racemic O-desmethylvenlafaxine | 110 | 1,500 | 7,500 | 76 | 1,350 | - |
| (+)-O-desmethylvenlafaxine | - | 1,700 | - | 23 | 1,920 | - |
| (-)-O-desmethylvenlafaxine | 85 | 430 | 5,700 | 62 | 385 | 5,390 |
| Duloxetine | | | | <1 | 5 | 499 |

| | | | | | | |
|---|---|---|---|---|---|---|
| - indicates less than 50% inhibition of functional reuptake at a 10 µM concentration. | | | | | | |

**Table 2: IC₅₀ and (Kᵢ) Values (nM) for Inhibition of Specific Binding in Radioligand Binding Assays**

| | **Transporter** | |
|---|---|---|
| **Compound** | **5-HT** | **NE** |
| (+)-O-DMV | 235 (38.3) | 10,600 (10,500) |
| SEP-227162 | 805(131) | 3,600 (3,570) |

The data displayed in Table 2 were obtained according to published methods. The IC₅₀ values (and calculated Kᵢ values) for inhibition of the binding of [³H]-paroxetine to the cloned human serotonin receptor expressed in HEK293 cells were determined according to Galli A, DeFelice LJ, Duke BJ, Moore KR, Blakely RD, "Sodium-dependent norepinephrine-induced currents in norepinephrine-transporter-transfected HEK-293 cells blocked by cocaine and antidepressants," Journal of Experimental Biology 198: 2197-2212 (1995). The IC₅₀ values (and calculated Kᵢ values) for inhibition of the binding of [¹²⁵I]-RTI-55 to the cloned human norepinephrine receptor expressed in MDCK cells were determined according to Shearman LP, McReynolds AM, Zhou FC, Meyer JS, "Relationship between [125I]RTI-55-labeled cocaine binding sites and the serotonin transporter in rat placenta," American Journal of Physiology 275: C1621-9 (1998).

All publications and patents mentioned herein are hereby incorporated by reference in their entirety as if each individual publication or patent was specifically and individually indicated to be incorporated by reference. In case of conflict, the present application, including any definitions herein, will control.

While specific embodiments have been discussed, the above specification is illustrative and not restrictive. Many variations will become apparent to those skilled in the art upon review of this specification and the claims below. The full scope of the invention should be determined by reference to the claims, along with their full scope of equivalents, and the specification, along with such variations.

The invention also relates to the following items:
1. A method of treating a sleep-related breathing disorder in a patient, comprising administering to a patient a therapeutically effective amount of O-desmethylvenlafaxine, or a pharmaceutically acceptable salt or solvate thereof.
2. The method of item 1, wherein the O-desmethylvenlafaxine is enriched for the (+) enantiomer.
3. The method of item 2, wherein the O-desmethylvenlafaxine is enriched for the (-) enantiomer.
4. The method of item 3, wherein the O-desmethylvenlafaxine is substantially free of (+)-O-desmethylvenlafaxine.
5. The method of item 1, wherein the O-desmethylvenlafaxine is administered conjointly with a second active agent, wherein said second active agent is a sleep enhancer, an agent that treats excessive daytime drowsiness, an anti-obesity agent, or an agent that stabilizes respiratory drive.
6. The method of item 1, wherein the O-desmethylvenlafaxine is administered conjointly with positive airway pressure treatment.
7. The method of item 1, wherein the O-desmethylvenlafaxine is administered orally, sublingually, or by oral inhalation.
8. A method of treating a sleep-related breathing disorder in a patient, comprising administering to a patient a therapeutically effective amount of duloxetine, or a pharmaceutically acceptable salt or solvate thereof.
9. The method of item 8, wherein the duloxetine is administered conjointly with a second active agent, wherein said second active agent is a sleep enhancer, an agent that treats excessive daytime drowsiness, an anti-obesity agent, or an agent that stabilizes respiratory drive.
10. The method of item 8, wherein the duloxetine is administered conjointly with positive airway pressure treatment.
11. The method of item 8, wherein the duloxetine is administered orally, sublingually, or by oral inhalation.
12. The method of item 8, wherein the sleep-related breathing disorder is obstructive sleep apnea.

## Claims

1. O-desmethylvenlafaxine, or a pharmaceutically acceptable salt or solvate thereof for use in treating a sleep-related breathing disorder.

2. The O-desmethylvenlafaxine of claim 1, wherein the O-desmethylvenlafaxine is enriched for the (+) enantiomer.

3. The O-desmethylvenlafaxine of claim 2, wherein the O-desmethylvenlafaxine is enriched for the (-) enantiomer.

4. The O-desmethylvenlafaxine of claim 3, wherein the O-desmethylvenlafaxine is substantially free of (+)-O-desmethylvenlafaxine.

5. The O-desmethylvenlafaxine of claim 1, wherein the O-desmethylvenlafaxine is prepared to be administered conjointly with a second active agent, wherein said second active agent is a sleep enhancer, an agent that treats excessive daytime drowsiness, an antiobesity agent, or an agent that stabilizes respiratory drive.

6. The O-desmethylvenlafaxine of claim 1, wherein the O-desmethylvenlafaxine is prepared to be administered conjointly with positive airway pressure treatment.

7. The O-desmethylvenlafaxine of claim 1, wherein the O-desmethylvenlafaxine is prepared to be administered orally, sublingually, or by oral inhalation.
